(19) Europäisches Patentamt
European Patent Office
Office européen des brevets

(11) EP 2 431 033 A1

(12) **EUROPEAN PATENT APPLICATION**

(43) Date of publication:
**21.03.2012 Bulletin 2012/12**

(51) Int Cl.:
***A61K 31/343*** *(2006.01)* ***A61P 9/04*** *(2006.01)*

(21) Application number: **10305879.8**

(22) Date of filing: **10.08.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **SANOFI**
**75013 Paris (FR)**

(72) Inventor: **The designation of the inventor has not yet been filed**

(74) Representative: **Bouron, Estelle et al**
**Sanofi-Aventis**
**Département Brevets**
**174, avenue de France**
**75013 Paris (FR)**

(54) **Dronedarone for preventing cardiovascular hospitalization or mortality in patients with lone atrial fibrillation**

(57) Use of dronedarone or pharmaceutically acceptable salts thereof, for the preparation of a medicament for the prevention of cardiovascular hospitalization or of mortality in patients with lone atrial fibrillation.



Printed by Jouve, 75001 PARIS (FR)

## Description

The present invention relates to the use of dronedarone or pharmaceutically acceptable salts thereof, for the preparation of a medicament for the prevention of cardiovascular hospitalization and/or of mortality in patients with lone atrial fibrillation.

2-n-Butyl-3-[4-(3-di-n-butylaminopropoxy)benzoyl]-5-methylsulphonamidobenzofuran, or dronedarone, and pharmaceutically acceptable salts thereof are described in European Patent EP 0 471 609 B1.

Dronedarone blocks potassium, sodium and calcium channels and also has anti-adrenergic properties.

Dronedarone is an anti-arrhythmic that is effective in maintaining sinus rhythm in patients presenting atrial fibrillation or atrial flutter.

The applicant has clinically proven that dronedarone significantly reduces cardiovascular hospitalizations and/or mortality in patients having a history of atrial fibrillation or of atrial flutter, in a safe and effective way.

In fact, the use of benzofuran derivatives to reduce post-infarction mortality in patients having a reduced left ventricular function after myocardial infarction, without any rhythm disorder requiring an anti-arrhythmic treatment, is known from Patent Applications WO 98/40067 and WO 97/34597.

In W02009/144550, dronedarone has been shown to reduce relevant clinical endpoints such as cardiovascular hospitalizations or death in patients with atrial fibrillation (AF) and additional risk factors and notably in patients with paroxysmal or persistent atrial fibrillation (AF) or atrial flutter (AFL), with a recent episode of AF/AFL and associated cardiovascular risk factors (i.e., age >70, hypertension, diabetes, prior cerebrovascular accident, left atrial diameter ≥50 mm or left ventricular ejection fraction [LVEF] <40%), who are in sinus rhythm or who will be cardioverted.

Atrial fibrillation (AF) affects about 2.3 million people in North America and 4.5 million people in the European Union and is emerging as a growing public health concern because of the aging of the population

AF is a condition in which the upper chambers of the heart beat in an uncoordinated and disorganized fashion, resulting in a very irregular and fast rhythm (i.e., an irregularly, irregular heartbeat). When blood is not completely pumped out of the heart's chambers, it can pool and clot. If a blood clot forms in the atria, exits the heart and blocks an artery in the brain, a stroke results. Consequently, about 15 percent of strokes result from AF.

AF is increasingly frequent with advancing age and is often caused by age-related changes in the heart, physical or psychological stress, agents that stimulate the heart, such as caffeine, or as a result of cardiovascular disease. The number is expected to double in the next 20 years. Without appropriate management, AF can lead to serious complications, such as stroke and congestive heart failure.

It is know that atrial fibrillation itself can cause changes in the electrical parameters of the heart known as electrical remodelling and in the structure of the cardiac chambers known as structural remodelling which tend to decrease the chances of the patient to get back into normal sinus rhythm. This vicious circle whereby "atrial fibrillation begets atrial fibrillation" has been well documented since the 1990s (Wijffels MC, Kirchhof CJ, Dorland R, Allessie MA.Atrial fibrillation begets atrial fibrillation. A study in awake chronically instrumented goats.Circulation. 1995 Oct 1;92(7): 1954-68.). It explains why when patients have been in atrial fibrillation for a long time they develop permanent atrial fibrillation with little or no chance to recover from this arrhythmia which becomes chronic.

Atrial fibrillation (AF) is the most commonly encountered cardiac rhythm disturbance. In the US, hospitalizations for AF have increased 2- to 3-fold in recent years [1] demonstrating the enormous economic burden of this arrhythmia. The most significant consequences of AF include congestive heart failure, a fivefold increase in the risk of stroke, and a twofold increase in mortality. The majority of the patients have cardiovascular comorbidities and other risk factors for hospitalization. There is, however, a subset of patients who have AF in the absence of structural heart disease and of extracardiac disorders. This condition is termed lone AF. In younger patients with lone AF, catheter ablation therapy has evolved as an established therapeutic approach. However, ablation therapy is difficult to perform, not widely available, and the rate of major complications is still significant. Therefore, pharmacological therapy remains an option even in these patients. Currently available antiarrhythmic drugs have limited efficacy in preventing AF recurrences, not all of them have rate-controlling properties in case of relapsing AF, and some are known to have unfavourable safety profiles. Dronedarone is a new multichannel blocking drug which has been demonstrated in the EURopean trial In atrial fibrillation or flutter patients receiving Dronedarone for the maintenance of Sinus rhythm (EURIDIS) and the American-Australian-African trial with DronedarONe In atrial fibrillation or flutter patients for the maintenance of Sinus rhythm (ADONIS) trials to be more effective than placebo in maintaining normal sinus rhythm after electrical, pharmacological or spontaneous conversion of AF/AFL and in controlling the ventricular rate during AF recurrences but with a comparable side effect profile to placebo. Moreover, in the ATHENA (A Placebo-Controlled, Double-Blind, Parallel Arm Trial to Assess the Efficacy of Dronedarone 400 mg bid for the Prevention of Cardiovascular Hospitalization or Death from Any Cause in Patients with Atrial Fibrillation/Atrial Flutter) trial, dronedarone has been shown to reduce important clinical endpoints such as cardiovascular hospitalizations or death in patients with AF and additional risk factors.

However it has not been studied whether similar beneficial effects can be observed in patients with idiopathic (lone) atrial fibrillation. Furthermore there is a paucity of data regarding the prevalence of lone AF in large samples of

AF patients and with respect to the associated incidence of major clinical events and about the need for recurrent hospitalization in patients with lone AF.

Lone atrial fibrillation may be defined as atrial fibrillation in patients without structural heart disease and without arterial hypertension (not treated with blood pressure lowering medications).

Thus, these applications neither disclose nor suggest the use of dronedarone to reduce cardiovascular hospitalizations and mortality in patients having lone atrial fibrillation or atrial flutter.

A subject of the present invention is therefore the use of dronedarone or a pharmaceutically acceptable salt thereof, for the preparation of a medicament, for the prevention of cardiovascular hospitalizations and/or of mortality in patients with lone atrial fibrillation.

A subject of the present invention is also the use of dronedarone or a pharmaceutically acceptable salt thereof, for the preparation of a medicament, taken twice a day with a meal, for the prevention of mortality and/or of cardiovascular hospitalizations notably in patients having lone atrial fibrillation.

More specifically, a subject of the present invention is the use of dronedarone or a pharmaceutically acceptable salt thereof, for the preparation of a medicament for the prevention of approximately 44% of cardiovascular hospitalizations or of mortality in patients having lone atrial fibrillation.

A subject of the present invention is also the use of dronedarone or a pharmaceutically acceptable salt thereof, for the preparation of a medicament for the prevention of cardiovascular hospitalizations in patients having lone atrial fibrillation.

More specifically, a subject of the present invention is also the use of dronedarone or a pharmaceutically acceptable salt thereof, for the preparation of a medicament for the prevention of approximately 46% of cardiovascular hospitalizations in patients having lone atrial fibrillation.

A subject of the present invention is also the use of dronedarone or a pharmaceutically acceptable salt thereof, for the preparation of a medicament for the prevention as mentioned above in patients having lone atrial fibrillation and at least one of the following medications:

- beta blocking agents,
- ACE inhibitors or A II receptor antagonists,
- oral anticoagulant,
- diuretics,
- statins,
- chronic antiplatelet therapy,
- calcium antagonists with heart rate lowering effects,
- digitalis,
- drugs interacting with the creatinine tubular secretion,
- moderate inhibitors of CYP3A4,
- NSAID.

NYHA means New York Heart Association.

Another subject of the invention is performed by providing dronedarone or pharmaceutically acceptable salts thereof, wherein said dronedarone or pharmaceutically acceptable salts thereof is provided along with information indicating that dronedarone or pharmaceutically acceptable salts thereof is indicated in patients having lone atrial fibrillation.

In an embodiment of the invention, the information comprises printed matter that advises that dronedarone or pharmaceutically acceptable salts thereof is indicated in patients having lone atrial fibrillation. In another embodiment, the printed material is a label.

The term "providing" includes selling, distributing, shipping, offering for sell, importing etc.

Mention may be made that "dronedarone for the prevention / treatment of " may be understood as use of dronedarone for the preparation of a medicament for use in the prevention / treatment of".

Thus another object of the invention is dronedarone or a pharmaceutically acceptable salt thereof for the prevention of cardiovascular hospitalizations or of mortality in patients with lone atrial fibrillation.

A subject of the present invention is also dronedarone or a pharmaceutically acceptable salt thereof for the prevention of mortality and/or of cardiovascular hospitalizations notably in patients having lone atrial fibrillation.

More specifically, a subject of the present invention is also dronedarone or a pharmaceutically acceptable salt thereof for the prevention of approximately 44% of cardiovascular hospitalizations or of mortality in patients having lone atrial fibrillation.

A subject of the present invention is also dronedarone or a pharmaceutically acceptable salt thereof, for the prevention of cardiovascular hospitalizations in patients having lone atrial fibrillation.

More specifically, a subject of the present invention is also dronedarone or a pharmaceutically acceptable salt thereof, for the prevention of approximately 46% of cardiovascular hospitalizations in patients having lone atrial fibrillation.

A subject of the present invention is also dronedarone or a pharmaceutically acceptable salt thereof, for the prevention as mentioned above in patients having lone atrial fibrillation and at least one of the following medications:

- beta blocking agents,
- ACE inhibitors or A II receptor antagonists,
- oral anticoagulant,
- diuretics,
- statins,
- chronic antiplatelet therapy,
- calcium antagonists with heart rate lowering effects,
- digitalis,
- drugs interacting with the creatinine tubular secretion,
- moderate inhibitors of CYP3A4,
- NSAID.

The invention also relates to a method of promoting the use of dronedarone or pharmaceutically acceptable salts thereof, the method comprising the step of conveying to a recipient at least one message selected from the group consisting of dronedarone or pharmaceutically acceptable salts thereof should be prescribed to a patient having lone atrial fibrillation.

The invention also concerns an article of manufacture comprising

a) a packaging material;
b) dronedarone or pharmaceutically acceptable salts thereof, and
c) a label or package insert contained within the packaging material indicating that dronedarone or pharmaceutically acceptable salts thereof is indicated in patients having lone atrial fibrillation.

The invention also relates to a package comprising dronedarone or pharmaceutically acceptable salts thereof and a label, said label comprising a printed statement which informs a prospective user that dronedarone or pharmaceutically acceptable salts thereof is indicated in patients having lone atrial fibrillation.

Another method of the invention comprises treating a patient having lone atrial fibrillation, said method comprising administrating to said patient a therapeutically effective amount of dronedarone, or a pharmaceutically acceptable salt thereof.

Another method of the invention relates to transforming a patient having lone atrial fibrillation by decreasing the patient's risk of cardiovascular hospitalizations or mortality, comprising administrating to said patient a therapeutically effective amount of dronedarone, or a pharmaceutically acceptable salt thereof.

A subject of the invention is a method of decreasing the risk of cardiovascular hospitalizations or mortality in a having lone atrial fibrillation, said method comprising administering dronedarone, or a pharmaceutically acceptable salt thereof.

In terms of clinical study, the prevention of "cardiovascular hospitalizations or of mortality" constitute what are referred to as composite criteria or a combined endpoint.

All the percentages given above correspond to average values.

Among the pharmaceutically acceptable salts of dronedarone, mention may be made of the hydrochloride.

The term "cardiovascular hospitalization" means a hospitalization which is caused by at least one of the following pathologies (Hohnloser et al., Journal of cardiovascular electrophysiology, January 2008, vol. 19, No. 1, pages 69-73):

- relating to atherosclerosis,
- myocardial infarction or unstable angina pectoris,
- stable angina pectoris or atypical thoracic pain,
- syncope,
- transient ischemic event or cerebral stroke (except intracranial haemorrhage),
- atrial fibrillation and other supraventricular rhythm disorders,
- non-fatal cardiac arrest,
- ventricular arrhythmia,
- cardiovascular surgery, except heart transplant,
- heart transplant,
- implantation of a cardiac stimulator (pacemaker), of an implantable defibrillator ("ICD") or of another cardiac device,
- percutaneous coronary, cerebrovascular or peripheral intervention,
- variations in arterial pressure (hypotension, hypertension, except syncope),

- cardiovascular infection,
- major bleeding/haemorrhage (requiring two or more blood cell pellets or any intracranial haemorrhage),
- pulmonary embolism or deep vein thrombosis,
- worsening of congestive heart failure including acute pulmonary oedema or dyspnoea from cardiac causes.

Consequently, the prevention of cardiovascular hospitalization may be understood as the prevention of cardiovascular hospitalization for at least one of the above mentioned pathologies.
Mention may be made of the prevention of cardiovascular hospitalization for atrial fibrillation and/or other supraventricular rhythm disorders.
Thus, a subject of the present invention is also the use of dronedarone or a pharmaceutically acceptable salt thereof, for the preparation of a medicament for the prevention of cardiovascular hospitalization for at least one of the above mentioned pathologies in patient with lone atrial fibrillation.
The term "mortality" or "death" are equivalent and cover mortality due to any cause, whether cardiovascular or non-cardiovascular or unknown.
The term "cardiovascular mortality" covers, in the context of the invention, mortality due to any cardiovascular causes (any death except those due to a non-cardiovascular cause), in particular death from an arrhythmic cause, also called arrhythmic death, and more particularly, sudden death from cardiovascular causes, also called sudden death or sudden cardiac death.
Cardiovascular mortality may be due for example to :

- Aortic dissection/aneurysm
- Cardiac tamponade
- Cardiogenic shock
- congestive heart failure
- Death during a cardiovascular transcutaneous interventional procedure or cardiovascular surgical intervention
- Hemorrhage (except cardiac tamponade)
- Myocardial infarction or unstable angina (including complications of myocardial infarction, except arrhythmias)
- Pulmonary or peripheral embolism
- Stroke
- Sudden cardiac death (eg, unwitnessed death or documented asystole)
- Ventricular arrhythmia, subclassified as torsades de pointes, ventricular extrasystole, ventricular fibrillation, ventricular tachycardia (non-sustained and sustained ventricular tachycardia), or other ventricular arrhythmia
- Unknown cause

Of course, it may be understood that "prevention of cardiovascular hospitalization and/or mortality" results in the reduction of the risk of cardiovascular hospitalization and or mortality or in the reduction of the need of cardiovascular hospitalization and or mortality.
Among the patients, mention may also be made of patients also exhibiting at least one of the following risk factors:

- age notably equal to or above 70, or even above 75,
- diabetes,
- history of cerebral stroke or of systemic embolism, i.e. prior cerebrovascular accident,
- left atrial diameter greater than or equal to 50 mm measured for example by echocardiography,
- left ventricular ejection fraction less than 40%, measured for example by two-dimensional echography.

The above mentioned risks factors may be defined as cardiovascular risk factors which are associated to atrial fibrillation.
Among the patients, notably patients having lone atrial fibrillation, mention may also be made of patients also exhibiting additional risk factors, i.e. at least one of the following pathologies:

- tachycardia,
- coronary disease,
- non-rheumatic heart valve disease,
- dilated cardiomyopathy of ischemic origin,
- ablation of atrial fibrillation or flutter, for example catheter ablation or endomyocardial ablation,
- supraventricular tachycardia other than atrial fibrillation or flutter,
- history of heart valve surgery,
- non-ischemic dilated cardiomyopathy,

- hypertrophic cardiomyopathy,
- rheumatic valve disease,
- sustained ventricular tachycardia,
- congenital cardiopathy,
- ablation, for example catheter ablation, for tachycardia other than for atrial fibrillation or flutter,
- ventricular fibrillation,

and/or at least one cardiac device chosen from:

- a cardiac stimulator,
- an implantable defibrillator ("ICD").

For their therapeutic use, dronedarone and pharmaceutically acceptable salts thereof are generally introduced into pharmaceutical compositions.

These pharmaceutical compositions contain an effective dose of dronedarone or of a pharmaceutically acceptable salt thereof, and also at least one pharmaceutically acceptable excipient.

Said pharmaceutical composition may be given once or twice a day with food.

The dose of dronedarone administered per day, orally, may reach 800 mg, taken in one or more intakes, for example one or two.

More specifically, the dose of dronedarone administered may be taken with food.

More specifically, the dose of dronedarone administered per day, orally, may reach 800 mg, taken in two intakes with a meal.

The dose of dronedarone administered per day, orally may be taken at a rate of twice a day with a meal for example with the morning and the evening meal.

More specifically, the two intakes may comprise same quantity of dronedarone.

There may be specific cases where higher or lower dosages are appropriate; such dosages do not depart from the context of the invention. According to the usual practice, the dosage appropriate for each patient is determined by the physician according to the method of administration, the weight, the pathology, the body surface, the cardiac output and the response of said patient.

Said excipients are chosen according to the pharmaceutical form and the method of administration desired, from the usual excipients which are known to those skilled in the art.

In said pharmaceutical compositions for oral, sublingual, subcutaneous, intramuscular, intravenous, topical, local, intratracheal, intranasal, transdermal or rectal administration, dronedarone, or the salt thereof, can be administered in unit administration form, as a mixture with conventional pharmaceutical excipients, to animals and to humans in the cases mentioned above.

The suitable unit administration forms comprise forms for oral administration, such as tablets, soft or hard gel capsules, powders, granules and oral solutions or suspensions, sublingual, buccal, intratracheal, intraocular or intranasal administration forms, forms for administration by inhalation, topical, transdermal, subcutaneous, intramuscular or intravenous administration forms, rectal administration forms, and implants. For topical application, dronedarone and pharmaceutically acceptable salts thereof can be used in creams, gels, ointments or lotions.

By way of example, a unit administration form of dronedarone or a pharmaceutically acceptable salt thereof, in tablet form, may correspond to one of the following examples:

| Ingredients | mg | % |
|---|---|---|
| Dronedarone hydrochloride (corresponding to 400 mg of base) | 426 | 65.5 |
| Methylhydroxypropylcellulose | 21.1 | 3.25 |
| Lactose monohydrate | 46.55 | 7.2 |
| Maize starch | 45.5 | 7 |
| Polyvinylpyrrolidone | 65 | 10 |
| Poloxamer 407 | 40 | 6.15 |
| Anhydrous colloidal silica | 2.6 | 0.4 |
| Magnesium stearate | 3.25 | 0.5 |
| | 650 | 100 |

| Ingredients | mg | % |
|---|---|---|
| Dronedarone hydrochloride (corresponding to 400 mg of base) | 426 | 65.5 |
| Microcrystalline cellulose | 65 | 10 |
| Anhydrous colloidal silica | 2.6 | 0.4 |
| Anhydrous lactose | 42.65 | 6.6 |
| Polyvinylpyrrolidone | 13 | 2 |
| Poloxamer 407 | 40 | 6.15 |
| Macrogol 6000 | 57.5 | 8.85 |
| Magnesium stearate | 3.25 | 0.5 |
| | 650 | 100 |

| Ingredients | mg |
|---|---|
| Dronedarone hydrochloride (corresponding to 400 mg of base) | 426 |
| Microcrystalline cellulose | 26 |
| Maize starch | 45.5 |
| Polyvinylpyrrolidone | 65 |
| Poloxamer 407 | 40 |
| Anhydrous colloidal silica | 3.25 |
| Magnesium stearate | 3.25 |
| Lactose monohydrate | 41.65 |
| | 650 |

| Ingredients | mg |
|---|---|
| Dronedarone hydrochloride (corresponding to 400 mg of base) | 213 |
| Microcrystalline cellulose | 13 |
| Maize starch | 22.75 |
| Polyvinylpyrrolidone | 32.5 |
| Poloxamer 407 | 20 |
| Anhydrous colloidal silica | 1.3 |
| Magnesium stearate | 1.625 |
| Lactose monohydrate | 20.825 |
| | 650 |

According to another of its aspects, the present invention also relates to a method for treating the pathologies indicated above, which comprises the administration, to a patient having lone atrial fibrillation, of an effective dose of dronedarone or a pharmaceutically acceptable salt thereof.

One method of the invention includes decreasing the risk of mortality, cardiac hospitalizations, or the combination thereof in a patient having lone atrial fibrillation, said method comprising administering to said patient an effective amount of dronedarone or a pharmaceutically acceptable salt thereof, with food.

In some embodiments of the above methods according to the invention, the administration of dronedarone or

pharmaceutically acceptable salt thereof prevents cardiovascular hospitalizations. In some embodiments of the above methods according to the invention, said patient also exhibits one or more of the above mentioned associated risk factors.

The present invention is illustrated by data from a post-hoc analysis of patients with lone atrial fibrillation enrolled in the clinical trials called EURIDIS, ADONIS and ATHENA trials with the following endpoints:

- cardiovascular hospitalization or death from any cause
- cardiovascular hospitalization
- all cause mortality
- cardiovascular mortality

In these trials, dronedarone has been shown to be effective for the prevention of mortality and/or morbidity (cardiovascular hospitalization) in patients with lone atrial fibrillation.

The studies designs comprising patients selection, inclusion and exclusion criteria, duration and treatment are described in Hohnloser and al., New England Journal of Medecine, 2009, 360:668-678 and Singh et al., New England Journal of Medecine, 2007, 357:987-999.

**I. Patient and methods**

**Patient population and procedures**

Individual data from patients with lone AF enrolled in the EURIDIS, ADONIS and ATHENA trials were entered in a central database. (SAS version 8.2). The institutional review board at each site approved the study, and all patients gave written informed consent for taking part in the studies. In short, common enrolment criterion for these studies was the presence of paroxysmal or persistent AF. Patients with permanent AF or with unstable hemodynamic conditions were excluded from participation.

Lone AF was defined as AF occurring in patients without structural heart disease or arterial hypertension hypertension (not treated with blood pressure lowering medications), and without extracardiac diseases associated with AF (i.e. thyroid disorders).

In all 3 studies, patients were randomly assigned to double-blind treatment with dronedarone 400 mg BID or matching placebo. Hospitalisation was defined as at least one overnight stay. The main outcome measure of this post-hoc analysis was the time to the composite endpoint of first hospitalization for cardiovascular reasons or death from any cause, and their individual components.

**Statistical methods**

Baseline parameters of patients with and without lone AF were compared with logistic regression analysis for qualitative and an ANOVA test for quantitative variables. All outcomes were estimated with the use of the Kaplan-Meier method and compared by means of the log-rank test. The relative risks and 95% confidence intervals of the composite endpoint and their individual components were calculated separately in patients with and without lone AF. All P values are two-tailed, and statistical significance was assumed at a p-value of 0.05 or smaller.

**II. Results**

The results obtained in this trial were analysed by the Kaplan Meier method for the figures, and the relative risk (RR) was estimated using Cox's proportional-effect regression model.

The relative risk (RR) is the ratio of the rates of occurrence of a hospitalization or of a death among the patients on dronedarone, relative to the patients on placebo.

The percentage reduction x of a given event (hospitalization, death, cardiovascular death, etc.) is calculated in the following way:

- All-cause mortality was not different in the two patient groups (HR: 1.02; 95%CI=0.31-3.43, p=0.885).
- All findings were homogeneous across studies and similar to those observed in the overall population.
- The adverse event profile of D in lone AF patients was similar to the one observed in the overall population. Patients with lone AF had similar drug discontinuation rates due to adverse events in the dronedarone and in the placebo arm.

**Patient population**

The 3 randomized clinical trials comprised a total of 5845 AF patients. Of those, 432 patients (192 on placebo, 240 on dronedarone) met the definition lone AF (prevalence 7%) (see table 1). The effect was homogeneous across the 3 studies contributing to the present analysis.

Table 1: Unadjusted analysis of time from randomization to first cardiovascular hospitalization or death from any cause in patients with lone AF

| | **Number of patients with endpoint (lone AF)** | | |
|---|---|---|---|
| **Studies** | **Placebo** | **Dronedarone 400mg BID** | **Relative Risk [95% CI] (a)** |
| EURIDIS | 6 (N=53) | 7 (N=81) | 0.681 [0.229;2.030] |
| ADONIS | 7 (N=50) | 1 (N=64) | 0.092 [0.011;0.747] |
| ATHENA | 33 (N=89) | 26 (N=95) | 0.654 [0.391;1.094] |
| Lone AF population (b) | 46 (N=192) | 34 (N=240) | 0.562 [0.361;0.877] |
| (a) Determined from Cox regression model<br>(b) Relative Risk from Cox model is adjusted on studies | | | |

Baseline clinical characteristics are depicted in Table 2. The mean age was 64±13 years, the mean systolic blood pressure 127 mmHg, 51% received OACs, 33% antiplatelet therapy, 36% betablockers, 15% digitalis and 10 % calcium antagonists (all medications given for rate control). Compared to the remaining patients, patients with lone AF were younger, had lower blood pressure, and received less cardiovascular medications. The average study follow-up time of patients with lone AF was 13.8 ± 7.2 months.

Table 2: Baseline characteristics in patients with or without lone AF at baseline

| | **Patients with lone AF at baseline** | **Patients without lone AF at baseline** | **P-value** |
|---|---|---|---|
| **Number of Patients** | **N= 432** | **5413** | |
| Age (years) | 63.5 (13.3) | 70.3 (9.6) | <0.001 |
| Male gender (%) | 300 / 432 (69.4 %) | 3005 / 5413 (55.5 %) | 0.001 |
| Heart rate | 63.6 (10.2) | 64.5 (10.6) | 0.183 |
| Systolic blood pressure | 127.0 (14.7) | 134.3 (17.7) | <0.001 |
| Diastolic blood pressure | 76.3 (9.0) | 78.2 (10.5) | <0.001 |
| Hypercholesterolemia (%) | 70 / 432 (16.2 %) | 2171 / 5413 (40.1 %) | <0.001 |
| NIDDM/IDDM (%) | 29 / 432 (6.7 %) | 1068/5413(19.7%) | <0.001 |
| Chronic renal failure (%) | 3 / 432 (0.7 %) | 171/5413(3.2%) | NC |
| CHF (%) | 0 / 432 (0.0 %) | 1580 / 5413 (29.2 %) | NC |
| CHF NYHA class III (%) * | 0 / 0 (.%) | 200 / 1580 (12.7 %) | NC |
| LVEF <35% (%) | 0 / 427 (0.0 %) | 218 / 5297 (4.1 %) | NC |
| Medication at baseline | | | |
| Beta blocking agents | 155 (35.9 %) | 3666 (67.7 %) | <0.001 |
| ACE inhibitors or A II preceptor antagonists | 0 (0.0 %) | 3748 (69.2 %) | NC |
| Oral anticoagulant | 221 (51.2 %) | 3339 (61.7 %) | 0.907 |
| Diuretics | 0 (0.0%) | 3063 (56.6%) | NC |
| Statins | 65 (15.0 %) | 3724 (68.8 %) | <0.001 |

(continued)

| | Patients with lone AF at baseline | Patients without lone AF at baseline | P-value |
|---|---|---|---|
| **Number of Patients** | **N= 432** | **5413** | |
| Chronic antiplatelet therapy | 141 (32.6 %) | 2416 (44.6 %) | 0.016 |
| Calcium antagonists with heart rate lowering effects | 43 (10.0 %) | 749 (13.8 %) | 0.055 |
| Digitalis | 66 (15.3 %) | 782 (14.4 %) | 0.616 |
| Drugs interacting with the creatinine tubular secretion | 13 (3.0 %) | 609 (11.3%) | 0.002 |
| Moderate inhibitors of CYP3A4 | 36 (8.3 %) | 560 (10.3 %) | 0.048 |
| NSAID | 30 (6.9 %) | 315 (5.8 %) | 0.124 |
| NIDDM means Non Insulin Dependant Diabetes Mellitus, IDDM means Insulin Dependent Diabetes Mellitus<br>*NYHA Class III vs Class I or II 1<br>NC : Non-convergence of the logistic regression<br>P-value comparing Lone AF population to non-Lone AF population using a logistic regression for qualitative variables and an ANOVA for quantitative variables, both adjusted on study | | | |

**Results for endpoints**

D led to a 44% reduction of cardiovascular hospitalizations or death (hazard ratio (HR)=0.56; 95%CI=0.36-0.88, p=0.004) and to a 46% reduction in cardiovascular hospitalizations alone (HR=0.54; 95%CI=0.34-0.87, p=0.004) compared to placebo (see scheme 1)).

In others words, dronedarone when added to standard of care including rate control, leads to a 44% reduction of the risk of CV hospitalizations or death in patients with lone AF (compared with a 23% reduction in patients with other forms of AF).

Scheme 1: Relative risks (dronedarone 400 mg BID versus placebo) and 95% confidence intervals of primary and secondary endpoints according to lone AF

| Characteristic | N | HR [95% CI] (a) | P-value (b) |
|---|---|---|---|
| **Cardiovascular hospitalization or death** | 5865 | 0.76 [0.69;0.84] | |
| With Lone AF | 432 | 0.56 [0.36;0.88] | |
| Without Lone AF | 5413 | 0.77 [0.70;0.85] | 0.12 |
| **Cardiovascular hospitalization** | 5865 | 0.75 [0.68;0.82] | |
| With Lone AF | 432 | 0.54 [0.34;0.87] | |
| Without Lone AF | 5413 | 0.76 [0.69;0.84] | 0.12 |
| **Death** | 5865 | 0.85 [0.67;1.08] | |
| With Lone AF | 432 | 1.02 [0.31;3.34] | |
| Without Lone AF | 5413 | 0.84 [0.66;1.07] | 0.78 |
| **Cardiovascular death** | 5865 | 0.69 [0.50;0.94] | |
| With Lone AF | 432 | 1.27 [0.21;7.63] | |
| Without Lone AF | 5413 | 0.67 [0.49;0.92] | 0.47 |

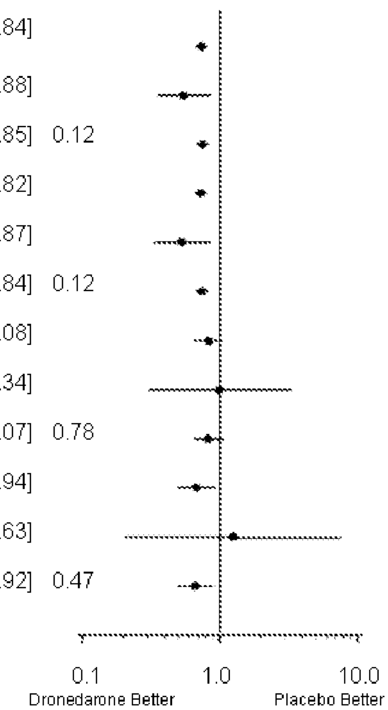


a: Determined from Cox regression model, adjusted on studies
b: P-value of interaction between baseline lone AF and treatment based on Cox regression model, adjusted on studies

The risk for cardiovascular hospitalizations or death in the placebo group after 1 year was 25% in the lone atrial fibrillation group compared to 29% the rest of the population (see table 3).

Table 3: Unadjusted analysis of time from randomization to first cardiovascular hospitalization or death from any cause in patients with lone AF:

| | **Placebo** | **Dronedarone 400mg BID** |
|---|---|---|
| | **(N= 192)** | **(N= 240)** |
| [Number of events, n | 46 | 34 |
| Cumulative incidence of events at 6 months [95% CI] | 0.182 [0.125 ; 0.240] | 0.057 [0.027 ; 0.087] |
| Cumulative incidence of events at 1 year [95% CI] | 0.249 [0.183 ; 0.315] | 0.128 [0.082 ; 0.173] |
| Cumulative incidence of events at 2 years [95% CI] | 0.316 [0.227 ; 0.404] | 0.230 [0.146; 0.313]] |
| Log-rank test p-value | 0.0039 | |
| Relative risk [95% CI]a | 0.562 [0.361; 0.877] | |

Patients with lone AF have a 23% risk for CV hospitalization in the first year, compared with 28% in patients

without lone AF (see table 4).

Table 4: Secondary Endpoint: Unadjusted analysis of time from randomization to first cardiovascular hospitalization in patients with or without lone AF

| | **Patients with lone AF at baseline** | | **Patients without lone AF at baseline** | |
|---|---|---|---|---|
| | **Placebo** | **Dronedarone 400mg BID** | **Placebo** | **Dronedarone 400mg BID** |
| | **(N= 192)** | **(N= 240)** | **(N= 2532)** | **(N= 2881)** |
| Number of events, n | 42 | 30 | 875 | 748 |
| Cumulative incidence of events at 6 months [95% CI] | 0.165 [0.110 ; 0.220] | 0.057 [0.027 ; 0.087] | 0.188 [0.173 ; 0.203] | 0.135 [0.122;0.148] |
| Cumulative incidence of events at 1 year [95% CI] | 0.234 [0.168 ; 0.299] | 0.113 [0.070 ; 0.156] | 0.279 [0.262 ; 0.297] | 0.208 [0.193 ; 0.224] |
| Cumulative incidence of events at 2 years [95% CI] | 0.285 [0.200 ; 0.370] | 0.195 [0.121 ; 0.269] | 0.393 [0.371 ; 0.415] | 0.321 [0.299 ; 0.343] |
| Log-rank test p-value | 0.0041 | | 12E-10 | |
| Relative risk [95% CI]a | 0.543 [0.339; 0.869] | | 0.758 [0.687; 0.836] | |
| a Determined from cause-specific Cox regression model adjusted on studies | | | | |

The majority of cardiovascular hospitalisations represented hospitalisations for treatment of AF or other supraventricular arrhythmias. In the placebo group 31 of 42 cardiovascular hospitalisations were for AF or other supraventricular arrhythmias compared to 16 of 30 in the dronedarone group.

The adverse event profile of dronedarone in lone AF patients was similar to the one observed in the patients not classified as lone AF at baseline. No difference was observed in the risks for adverse events between placebo and dronedarone independent from the AF type. Patients with lone AF had similar drug discontinuation rate in the dronedarone and in the placebo arm (Scheme 2), however drug discontinuation happened more often in the dronedarone arm in the patients with other forms of AF.

Scheme 2: Relative risks (dronedarone 400 mg BID versus placebo) and 95% confidence intervals of adverse events according to lone AF.

| Endpoints | N | HR [95% CI] (a) | P-value (b) |
|---|---|---|---|
| **TEAE** | | | |
| Overall | 5841 | 1.15 [1.08;1.22] | |
| Lone AF | 432 | 1.04 [0.83;1.32] | |
| No lone AF | 5392 | 1.15 [1.08;1.23] | 0.37 |
| **Serious TEAE** | | | |
| Overall | 5841 | 0.92 [0.82;1.04] | |
| Lone AF | 432 | 0.84 [0.50;1.42] | |
| No lone AF | 5392 | 0.92 [0.82;1.04] | 0.51 |
| **AE leading to IP discontinuation** | | | |
| Overall | 5841 | 1.58 [1.33;1.87] | |
| Lone AF | 432 | 1.29 [0.69;2.41] | |
| No lone AF | 5392 | 1.58 [1.33;1.89] | 0.47 |

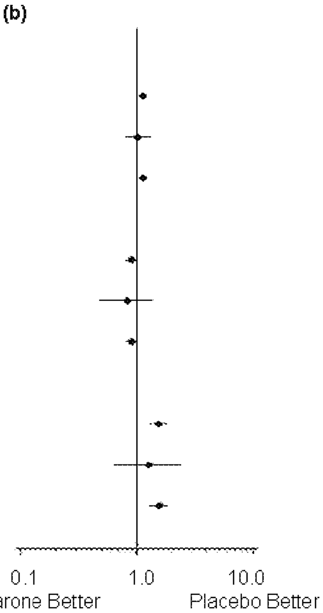


TEAE: treatment emergent adverse event; AE: adverse event; IP: Investigational product

a: Determined from Cox regression model, adjusted on studies
b: P-value of interaction between baseline lone AF and treatment based on Cox regression model, adjusted on studies.

## Claims

1. Dronedarone or a pharmaceutically acceptable salt thereof, for the prevention of cardiovascular hospitalizations and/or of mortality in patients with lone atrial fibrillation.

2. Dronedarone or a pharmaceutically acceptable salt thereof according to claim 1, for the prevention of approximately 44% of cardiovascular hospitalizations or of mortality in patients having lone atrial fibrillation.

3. Dronedarone or a pharmaceutically acceptable salt thereof to claim 1, for the prevention of cardiovascular hospitalizations in patients having lone atrial fibrillation.

4. Dronedarone or a pharmaceutically acceptable salt thereof to claim 1, for the prevention of approximately 46% of cardiovascular hospitalizations in patients having lone atrial fibrillation.

5. Dronedarone or a pharmaceutically acceptable salt thereof to claim 1, for the prevention as mentioned above in patients having lone atrial fibrillation and at least one of the following medications:

- beta blocking agents,
- ACE inhibitors or A II I receptor antagonists,
- oral anticoagulant,
- diuretics,
- statins,
- chronic antiplatelet therapy,
- calcium antagonists with heart rate lowering effects,
- digitalis,
- drugs interacting with the creatinine tubular secretion,
- moderate inhibitors of CYP3A4,
- NSAID.

6. Method of providing dronedarone or pharmaceutically acceptable salts thereof, wherein said dronedarone or pharmaceutically acceptable salts thereof is provided along with information indicating that dronedarone or pharmaceutically acceptable salts thereof is indicated in patients having lone atrial fibrillation.

7. Method according to claim 6, wherein the information comprises printed matter that advises that dronedarone or pharmaceutically acceptable salts thereof is indicated in patients having lone atrial fibrillation.

8. Method according to claim 7, wherein the printed material is a label.

9. Method of promoting the use of dronedarone or pharmaceutically acceptable salts thereof, the method comprising the step of conveying to a recipient at least one message selected from the group consisting of dronedarone or pharmaceutically acceptable salts thereof should be prescribed to a patient having lone atrial fibrillation.

10. Article of manufacture comprising:

a) a packaging material;
b) dronedarone or pharmaceutically acceptable salts thereof, and
c) a label or package insert contained within the packaging material indicating that dronedarone or pharmaceutically acceptable salts thereof is indicated in patients having lone atrial fibrillation.

11. Package comprising dronedarone or pharmaceutically acceptable salts thereof and a label, said label comprising a printed statement which informs a prospective user that dronedarone or pharmaceutically acceptable salts thereof is indicated in patients having lone atrial fibrillation.

12. Method of treatment of a patient having lone atrial fibrillation, said method comprising administrating to said patient a therapeutically effective amount of dronedarone, or a pharmaceutically acceptable salt thereof.

13. Method of transforming a patient having lone atrial fibrillation by decreasing the patient’s risk of cardiovascular hospitalizations or mortality, comprising administrating to said patient a therapeutically effective amount of dronedarone, or a pharmaceutically acceptable salt thereof.

Europäisches Patentamt
European Patent Office
Office européen des brevets

# EUROPEAN SEARCH REPORT

Application Number
EP 10 30 5879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YALTA KENAN ET AL: "Dronedarone: a promising alternative for the management of atrial fibrillation." October 2009 (2009-10), CARDIOVASCULAR DRUGS AND THERAPY / SPONSORED BY THE INTERNATIONAL SOCIETY OF CARDIOVASCULAR PHARMACOTHERAPY OCT 2009 LNKD- PUBMED:19669399, VOL. 23, NR. 5, PAGE(S) 385 - 393 , XP8127681 ISSN: 1573-7241 * abstract * * Introduction [0001] * ----- | 6-8,10, 11 | INV. A61K31/343 A61P9/04 |
| X,D | WO 2009/144550 A2 (SANOFI AVENTIS [FR]; GAUDIN CHRISTOPHE [FR]; HAMDANI NACERA [FR]; RADZ) 3 December 2009 (2009-12-03) * claim 71 * ----- | 6-8,10, 11 | |
| X | HOHNLOSER STEFAN H ET AL: "Effect of dronedarone on cardiovascular events in atrial fibrillation." THE NEW ENGLAND JOURNAL OF MEDICINE 12 FEB 2009 LNKD- PUBMED:19213680, vol. 360, no. 7, 12 February 2009 (2009-02-12), pages 668-678, XP002604030 ISSN: 1533-4406 * abstract * * table 1 * ----- | 6-8,10, 11 | TECHNICAL FIELDS SEARCHED (IPC) A61K |

The present search report has been drawn up for all claims

2

| Place of search | Date of completion of the search | Examiner |
|---|---|---|
| The Hague | 8 October 2010 | Strack, Eberhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 30 5879

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2010

| Patent document cited in search report | Publication date | Patent family member(s) | Publication date |
| --- | --- | --- | --- |
| WO 2009144550 A2 | 03-12-2009 | PE 18092009 A1 | 03-12-2009 |
| | | US 2010048694 A1 | 25-02-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0471609 B1
- WO 9840067 A
- WO 9734597 A
- WO 2009144550 A


### Non-patent literature cited in the description


- **WIJFFELS MC ; KIRCHHOF CJ ; DORLAND R ; ALLESSIE MA.** Atrial fibrillation begets atrial fibrillation. A study in awake chronically instrumented goats. *Circulation,* 01 October 1995, vol. 92 (7), 1954-68
- **HOHNLOSER et al.** *Journal of cardiovascular electrophysiology,* January 2008, vol. 19 (1), 69-73
- **HOHNLOSER.** *New England Journal of Medecine,* 2009, vol. 360, 668-678
- **SINGH et al.** *New England Journal of Medecine,* 2007, vol. 357, 987-999